# EUROPEAN PATENT SPECIFICATION

(11) **EP 1 372 560 B1**
(45) Date of publication and mention of the grant of the patent: **03.05.2006**
(21) Application number: 01944015.5
(22) Date of filing: 15.06.2001
(51) Int. Cl.: A61F 13/60

(54) **MECHANICAL TAPE FASTENING SYSTEM FOR DISPOSABLE ABSORBENT ARTICLES**
MECHANISCHES KLEBEBANDBEFESTIGUNGSSYSTEM FÜR SAUGFÄHIGE EINWEGARTIKEL
SYSTEME MECANIQUE DE FIXATION PAR BANDE ADHESIVE POUR ARTICLES ABSORBANTS JETABLES

(30) Priority: 22.01.2001 SE 0100173
(43) Date of publication of application: 02.01.2004
(73) Proprietor: SCA Hygiene Products AB, 405 03 Göteborg (SE)
(72) Inventor: KLING, Robert, S-511 63 Skene (SE); FORGAR, Monica, S-417 28 Göteborg (SE); HEDEN, Anna, S-434 92 Vallda (SE)
(74) Representative: Romare, Laila Anette
(86) International application number: PCT/SE2001/001367
(87) International publication number: WO 2002/056814

(56) References cited:
- EP-A1- 0 520 087
- WO-A1-00/27329
- WO-A1-98/22069
- US-A- 3 554 195
- US-A- 5 019 073
- US-A- 6 142 986

## Description

### TECHNICAL FIELD

The present invention concerns a disposable absorbent article, such as an baby diaper, an incontinence diaper or the like, comprising a first end portion and a second end portion, which portions are intended completely or partly to enclose the user's waist area during use of the article, and an intermediate crotch portion, said portions comprising an inner layer, which is turned towards the user during use and which is liquid-permeable at least in the crotch portion opposite the user's genitals, a liquid-impermeable outer layer, and an absorbent body disposed between said outer and inner layers, said article being formed in the area of the second end portion with mechanical tape fasteners intended, upon application of the article, to be detachably interconnected with one or several fastening parts disposed on the outer layer of the first end portion and being complementary to said tape fasteners.

### BACKGROUND ART

Various types of fasteners have been used for attaching disposable absorbent articles, such as baby diapers, incontinence diapers or the like, around the user's waist. As examples of fasteners may be mentioned adhesive tape fasteners and mechanical tape fasteners. Adhesive fastener systems allowing tape ends to be repeatedly fastened, unfastened and refastened have been available on the market since the beginning of the 80s. One adhesive tape fastener system of this type is described in US 5 024 672.

In analogy with the expressions adhesive tape fastener systems and adhesive tape fasteners are used herein the expressions mechanical tape fastener systems and mechanical tape fasteners to define fastener systems and fasteners, wherein the bond is effected not by adhesive means but by means of mechanical interlocking between hooks disposed on one of the parts of a fastening known as a hook-and-loop fastener and loops, apertures or fibre filaments disposed on the other hook-and-loop fastener part.

In most respects, modern adhesive tape fastener systems function satisfactorily. A serious problem is, however the deteriorated quality of the adhesion, should for example talcum powder or baby oil be spilled onto the pressure-sensitive adhesive substance or onto the bonding face to which the tape is to be attached. This problem may be removed by using mechanical tape fastener systems. In addition, consumers want disposable absorbent articles that are soft to the touch and have a textile-like appearance as opposed to the plastic films of which earlier outer layers of disposable absorbent articles were predominantly formed. Hitherto, mechanical tape fastener systems have been too expensive to compete with adhesive tape fastener systems. In recent years novel mechanical tape fastener systems have, however been developed that are more price-competitive. This fact in conjunction with the increasing use of textile-like outer layers and elimination of the problems caused by spillage on the bonding faces have contributed to manufacturers of disposable absorbent articles using mechanical tape fastener systems more and more.

By applying mechanical tape fasteners on both sides of e.g. the rear end portion of a diaper and providing a complementary bonding face on the external face of the opposite end of the diaper in a manner corresponding to that described in the above patent specification, US 5 024 672, the problem of fastening of the diaper as it is being put on, unfastening and refastening thereof is solved in the principally corresponding manner. Such a mechanical tape fastener system is described in e.g. EP-A1-0324578. This publication also touches on the problem involved in sealing a used diaper such that it forms a closed package for reliable enclosure of faeces inside the diaper, and the publication describes one means of solving this problem.

The latter publication mentions the problem arising because the mechanical tape fastener may unintentionally hook onto parts of the diaper before attachment to the intended bonding face has been made.

Over the years, the permanent anchorage of tape fasteners has been subject to much development, and many suggested solutions have been presented in the patent literature. This is true also as concerns the problem of protecting the bonding face of the tape fasteners from unintentionally adhering where not wanted before the diaper is put on. The tape fasteners, which are anchored to the article by the producer in the process of the manufacture of the article, are exposed to considerable stress as the diaper is being put on and the forces arising in connection therewith are absorbed by the point of anchorage. If the tape fastener is attached to the external layer of the article only, there is a risk that this layer be torn as the article is being put on. This problem is discussed already in US 3 867 940, and the solution suggested in that patent specification is to reinforce the external layer in the area of the point of anchorage.

The solution most predominantly found on the market and also in the patent literature is the use of the so-called Y-tape, which comprises two branches that are applied about the edge portion of the diaper, with one branch on the external layer and the other on the inner layer, thus making use of the inherent strength of the inner as well as of the external layer. The above publication EP-A1-0324578 and WO 95/05140, for example, disclose a Y-tape designed for mechanical tape fasteners.

One disadvantage inherent in Y-tapes is that they have to be attached on both sides of an edge portion of a disposable absorbent article, such as a diaper, during manufacturing. The manufacturing takes place in the form of a web travelling at a very high speed, a feature that makes the application of Y-tapes very complex. A further disadvantage is that the position of tape fasteners, applied around the edge portion of the rapidly advancing web of articles, such as diapers, may lead to serious drawbacks, both as regards the freedom of changing the manufacturing process and the freedom of changing the article itself.

Another prior-art solution is the so-called Z-folded tape, which is anchored permanently to the external face of the outer layer of the article. A Z-folded tape is shown e.g. in US Patent Specification 4 576 598. A drawback inherent in Z-folded tapes is that they are attached to the external face of the outer layer of for instance a diaper, with the result that the Z-tape, once attached, is unprotected during the subsequent processing steps, said steps usually including cross-cutting, cross-folding and insertion in a bag. To some extent this is applicable also to diapers comprising Y-tapes.

In the storage condition of the article, i.e. before it is used, adhesive and hook-on fastening parts on the tape fasteners should be safely covered in order to prevent unintentional hook-on or adhesion, respectively, that makes handling of the article more difficult as the latter is being put on. Hitherto, this problem usually has been solved by ensuring that in the storage condition of the tape fasteners, adhesive parts on said fasteners, including on mechanical tape fasteners, abut against release-agent coated plies of material. A solution of this kind is described for instance in the above mentioned EP-A1-0 324 578. Release-agent coatings are, however, comparatively expensive while at the same time they often require the provision of an additional layer of material in the tape fastener, which makes the design of the latter more complex. When mechanical tape fastener systems are used, it is of course conceivable to arrange for the mechanical bonding faces of the tape fastener, in the storage condition of the latter, to abut against and be mechanically interconnected with a part of a hook-and-loop fastener that is complementary to said bonding face. If a safe interconnecting bond is wanted, a solution of this kind does, however require the provision of complementary bonding faces for all mechanical fasteners of the article, with resulting increased material costs for and a more complex manufacture of said article.

### DISCLOSURE OF INVENTION

By means of the present invention, the above problems found in absorbent articles of the kind defined in the introduction have been eliminated.

For this purpose, the article in accordance with the invention is characterised in that the article comprises at least two of said tape fasteners, which are arranged on the inner layer of the article at opposite side portions of said second end portion,
that the tape fasteners have one longitudinal and one transversal extension,
that said tape fasteners are permanently joined to the inner layer of the article by means of a producer's bonding face arranged on a first end part of the tape fasteners,
that a user's bonding face is arranged on the opposite second end part of the fasteners, the user's end part,
the tape fasteners are arranged in a folded storage condition, each one of said tape fasteners being formed with an odd number of folds and the bonding faces of both said producer's bonding face and said user's bonding face being turned towards the inner layer of the article,
that the user's end parts of two opposite tape fasteners face one another in said storage condition and that in the storage condition of said article individual fold parts are joined to at least one of the other fold parts by means of a breakable bond, in the form of thermal or ultrasonic welds, said bond being breakable in order to establish the position of use of said tape fasteners.

Because the mechanical tape fastener is attached to the inner face of the inner layer of the article, the manufacturing process may be performed in a simpler and more reliable manner than in the case of the prior-art solutions involving Y-tapes. All folding steps with regard to the mechanical tape fasteners for the article in accordance with the present invention may be performed at a considerable lower speed than at the high web velocities found in modern machines for rational and competitive production of disposable absorbent articles, such as baby diaper. Anchorage of the fully folded mechanical tape fastener in accordance with the present invention onto the web of articles travelling at a high speed may be effected in a more controlled way than when Y-tapes are used, which, when they are to be applied, need to be folded about the edge portion of individual articles. To attach the mechanical tape fasteners to the article in accordance with the teachings of the present invention only the producer's bonding face need to be applied against the inner layer and be connected to that layer, which from a production point of view is simpler and offers a larger degree of freedom as concerns production changes and replacements of the materials of which the article is constructed.

By the expression breakable bonds as used herein is to be understood bonds that do not require supplementary materials such as release coatings or an additional bonding zone designed solely for the storage condition of the tape fastener. Preferably, the bonds are in the form of thermally or ultrasonically produced welds.

The tape fasteners preferably are arranged in their entirety on the inner layer, with the laterally outermost end edges of the tape fasteners being located interiorly of the lateral edges of said lateral portions. In this manner, the tape fasteners are well protected during the subsequent processing steps, particularly in the case of the conventional hourglass-shaped disposable absorbent articles discussed herein, i.e. baby diaper, adults' diapers and the like, wherein the tape fasteners are arranged on lateral portions that are folded inwards prior to cross-cutting, cross-folding and insertion into packages.

Another suitable embodiment of the invention is characterised in that the mechanical tape fasteners comprise one fold only and in that the latter forms the laterally outermost end edge of the tape fastener in said storage condition, that the tape fasteners comprise a middle part, which by means of said breakable bond is connected to the first end part and which covers the latter, and in that when the user's bonding face is in said folded storage condition, the opposite end part is located laterally interiorly of the first end part.

Additional preferred embodiments will appear from the appended claims.

### BRIEF DESCRIPTION OF DRAWINGS

The invention will be described in closer detail in the following with reference to the accompanying drawings, wherein:

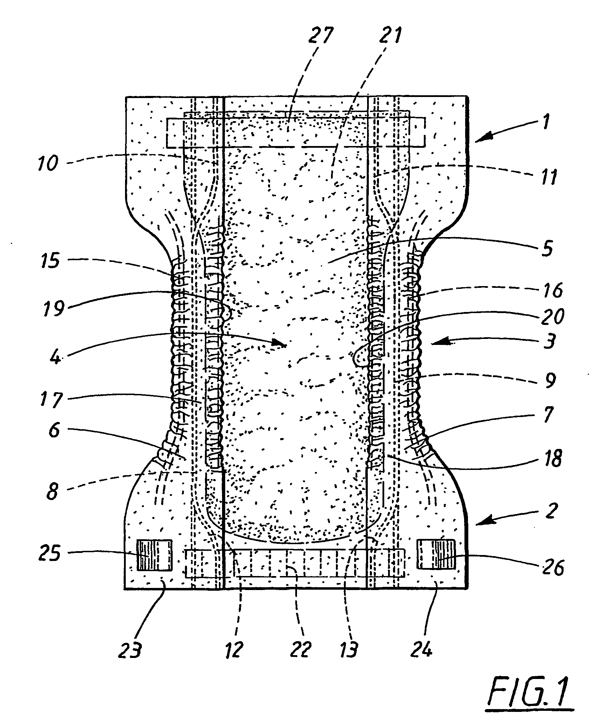

- Fig 1: shows a baby diaper and a flat, extended position and as seen from the inside towards the inner layer of the diaper.
- Fig 2: shows the baby diaper of Fig 1 in a flat, extended position as seen from the outside towards the outer layer of the diaper.
- Fig 3: is a view on an enlarged scale of a corner portion of the diaper of Fig 1, showing a mechanical tape fastener in condition of storage attached to the inner layer of the diaper.
- Fig 4: is a view of the corner portion of Fig 3, showing the tape fastener in the position of use.
- Fig 5: is a sectional view along line V-V of Fig 4, shown on a larger scale.
- Fig 6: is a schematic view of different types of bond ruptures obtained by means of a method of testing the strength required to break a bond in the tape fastener in the storage condition of the latter.

### MODES FOR CARRYING OUT THE INVENTION

The baby diaper illustrated in the drawing is hourglass-shaped, including a first end portion 1, a second end portion 2, and an intermediate crotch portion 3. The diaper comprises an inner layer 4 consisting of a longitudinally extending middle portion 5, the central area of which is liquid-permeable and which consists of e.g. a fibre cloth, a so-called non-woven fabric, and two longitudinally extending lateral edge portions 6, 7, which preferably are liquid-impermeable or hydrophobic. The middle portion 5 is connected to the lateral edge portions 6, 7 by means of interconnecting lines 8,9 and 10,11,12,13, said interconnecting lines preferably being in the form of ultrasonic welds. The lateral portions 6, 7 may consists of a non-woven fabric that has been treated to make it hydrophobic, or else it could consist of some other liquid-impermeable material known to the expert. The inner layer comprised of said portions is joined at least along its lateral and end edges to an external layer 14, which in the present case is formed in one piece from a liquid-impermeable material, such as a laminated product of polyethylene and a thin outer layer of a fibre cloth.

Along the edges of the crotch portion 3, the diaper is provided with leg-abutting elastic means 15, 16. The lateral portions 6, 7 overlap the middle portion somewhat laterally, forming inner barriers 17, 18 serving to prevent urine and faeces from spreading sideways in the diaper in the direction towards the diaper edges. The barriers 17, 18 are provided at their inner edges with pre-stressed elastic bands or threads 19, 20, which extend essentially along the crotch area of the diaper and which are arranged, when the diaper is in use, to make the barriers stand up from the inner layer. In the end portions of the diaper, the barriers are kept in a position of abutment against the middle portion 5 by means of the interconnecting lines 10-13. An absorbent body 21 is enclosed between the outer layer 14 and the inner layer 4. In accordance with the shown embodiment, the absorbent body 21 widens somewhat in the area of the first end portion 1, the front portion, and has a softly rounded configuration in the opposite end portion, the rear portion 2 of the diaper. The absorbent body 21 could consist for instance of cellulose fluff having mixed thereinto high absorbent materials in the form of particles or fibres.

At the rear portion 2 of the diaper, a pre-stressed elastic waistband 22 is provided between the outer and inner diaper layers.

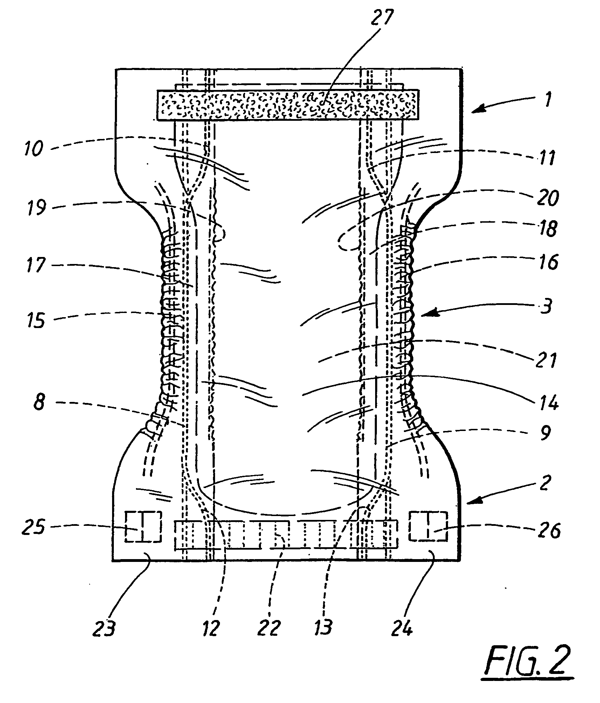

At the two opposite lateral portions 23, 24, viz. the corner portions of the rear diaper portion 2, the diaper is provided with mechanical tape fasteners 25, 26, which are permanently joined to the inner layer 4 by means of a producer's bonding face. At the opposite end portion 1 of the diaper, viz. the front portion, a bonding face, complementary to the mechanical tape fasteners, is arranged on the external face of the outer layer 14, as most clearly apparent from Fig 2. This bonding face is formed by an elongate band 27, serving as one of the parts of a fastening of the kind known as a hook-and-loop fastener, the other part of which is formed by a user's bonding face on the tape fasteners as will be described in closer detail in the following. The material of the elongate band 27 is formed with apertures, loops or fibre filaments for co-operation with hooks formed on the tape fastener. Examples of suitable materials for the elongate band are described in the above-mentioned patent specification EP-A1-0 324 578.

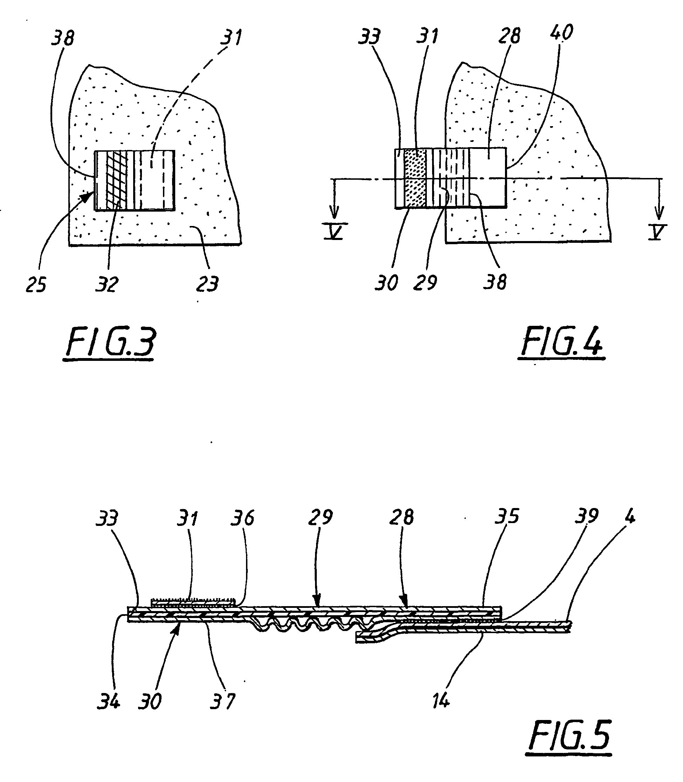

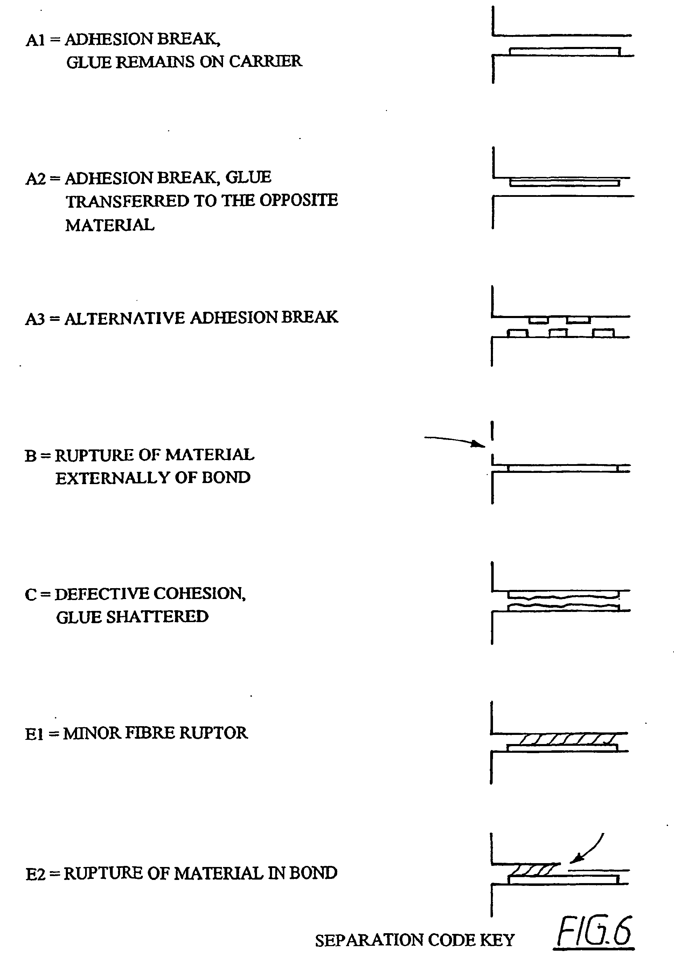

Fig 3 illustrates a mechanical tape fastener 25 as attached in its storage position to the inner layer of the corner portion 23. The tape fastener is prefabricated and is applied in folded condition, the single fold 38 forming the laterally outermost end edge of the tape fastener. Between the fold 38 or end edge and an edge 40, the fastener is formed with a producer's bonding face 28, see Fig 5, which is coated with an adhesive 39 for permanent anchorage of the tape fastener in the process of manufacture of the baby diaper. In accordance with the shown embodiment, the tape fastener comprises a support strip comprising three layers, which will be described in more detail with reference to Fig 5. The support strip essentially has three sub-parts, which are most clearly apparent from Figs 4 and 5. One sub-part is formed by the producer's bonding part 28 that extends between the fold 38 and the edge 40. In addition, there is a middle part 29 and a distal part 30. One of the parts of a hook-and-loop fastener is applied on that latter part, said part being a strip formed with a users' bonding face 31 in the form of hooks or the like, said hooks projecting from the strip and, upon use of the hook-and-loop fastener as the diaper is being put on about the waist of the user, being anchored in the elongate band 27 that forms the other part of the hook-and-loop fastener. Examples of a mechanical tape-attachment part formed with hooks or the like to form said users' bonding face 31 are shown in the previously mentioned Patent Specification EP-A1-0 324 578.

In its storage condition, the tape fastener 25 is folded as shown in Fig 3, and in this condition the middle part 29 is joined to the producer's part 28 by means of a bond in the form of thermal or ultrasonic welds. Fig 3 shows this bond 32 in the form of a pattern of linear welds. The advantage of this type of bond is that no additional material in the form of glue or release-agent coatings and associated support strips are needed. In the storage condition, the users' bonding face 31 including the hooks and similar means thereon is protected by the support strip of the tape fastener and there is no risk for unintentional hook-on of the bonding face before the bond 32 is broken. The inner layer of a baby diaper usually has a textile-like open fibrous face to which the bonding face 31 of the mechanical tape fastener adheres somewhat, and in the storage condition, the bonding face 31 adheres weakly to the inner diaper layer at the corner portion 23. The outer edge portion 33 externally of the bonding face 31 could alternatively be provided with a pressure-sensitive bonding agent (not shown), serving both to bind the distal part of the tape fastener stronger to the inner layer of the diaper in the storage condition of the latter and to seal the used diaper such that a package is formed enclosing urine and faeces inside the diaper.

The support strip of the tape fastener 25 is a three-ply strip as appears from Fig 5. An intermediate ply 34 in the form of an elastic film, which is elastically stretchable when exposed to stress in conjunction with the putting-on of the diaper and as the infant moves. Stress normally occurring in conjunction with the putting-on of the diaper is in the order to 200-2000g, the stress being higher on the tape fastener that is secured the last compared with that on the tape fastener that is secured first. Stress load arising during use of the diaper as a result of the movements of the infant is in the order of 100-500g, possibly somewhat higher in the case of chubby infants.

In accordance with the shown embodiment, a ply 35 of a non-woven fabric is laminated together with the elastic film. The non-woven ply follows the movements of the elastic film. The strip formed with the mechanical bonding face of the tape is joined to said non-woven ply35 by means of a bonding agent 36. A ply 37 of a non-elastic fabric is attached to the opposite face of the elastic film 34. By non-elastic as used in this case is to be understood that the ply 37 does not stretch when subjected to the stress to which the tape fastener is exposed during use of the diaper. Along the producer's bonding part 28 and along the distal part, the non-elastic non-woven ply is joined to the elastic film, with the result that the elastic capacity of the latter is lost in these areas. In the middle part 29, however, the non-elastic non-woven ply is corrugated and joined to the elastic film at spaced-apart points only, as shown in Fig 5. Consequently, the middle part of the tape fastener is elastically stretchable from the position of Fig 5 to the completely extended position, wherein the corrugations of the non-elastic non-woven ply are completely straightened-out. Fig 5 shows part pieces of the inner layer 4 and the external layer 14 of the diaper as well as a coat 39 of an adhesive coating, viz. the producer's bonding face, disposed between the tape fastener and the inner ply.

The bond 32 between the middle part 29 of the tape fastener and the producer's bonding part 28 should be of a magnitude ensuring reliable attachment of the mechanical tape fastener to the diaper in the folded tape-fastener position while at the same the bond should not be difficult to break for the person that is about to put on the diaper. Research has shown that the bond is too weak when forces below 0.2 N are sufficient to unfasten a strip having a width of 40 mm, whereas the bond is difficult to break, when forces above about 2.0 N are required to unfasten a strip having a width of 40mm. Thus, the bond functions satisfactorily, when a force in the range of from 0.2 to 2.0 is required to break the bond on a strip having a width of 40mm. Preferably, the required force to break the bond should range from 0.5 to 1.5 N for a strip having a width of 40 mm.

The measurement method for determination of the force necessary to break the bond will be described in the following.

### Testing method for measurement of lamination strength:

The object of this method is to determine the mean value of the force necessary to separate from one another two different sheets of a laminate. This method is applicable to laminates comprising two or several sheets that are joined together by gluing, thermo-bonding or welding.

### Principle

Separate the sheets and attach the individual sheets in a tensile strength tester and hold the non-separate part in a manner ensuring that delamination takes place at an angle of 90°.
- Test the material in the transverse and the machine directions.

### Equipment:

Tensile strength tester
Printer with plotter function
Punching or cutting equipment

### Preparation of specimens:

- Cut or punch specimens of 25x200 mm, five in the transverse direction and five in the machine direction. The strips should be evenly distributed over the entire specimen.
- Condition the specimens for 4-48 hours at an air humidity level of 50 ± 5% and at 23 ± 2°C.
- Wet specimens: Put the test strips in a sealable plastic bag. Pour deionised water into the bag and seal it. Leave it in a climate-controlled space for 4 hours. Do not wet more test strips at a time than can be delaminated within 30 minutes. In this manner, all test strips will have a "wet time" of between 4 and 4.5 hours.

### Procedure:

- Prepare the tensile-strength tester in accordance with the apparatus instructions
   Length of clip 50 mm
   Pulling speed 300 mm/min
   Speed of paper 300 mm/min
   Calibrate the tensile-strength tester
   Separate the sheets at one end of the test strip
   Attach the edges in the clips
   Support the laminated part of the specimen with one hand, loosely and at right angles to the laminated part, during the testing process
   Note the force during displacement of the draw-head over 250 mm, i.e. during delamination of a length of 125 mm
   In the case of glued laminates: Note when the delamination occurs and note the respective separation codes in accordance with the separation-code key shown in Fig 6
   Perform five acceptable tests in the cross-machine direction and five in the machine direction

### Calculate and note the results:

Calculate the mean force (N/25 mm) and the mean peak value (N/25 mm) of the movement of the draw-head from 10 mm to 210 mm.

The report is to include:
- The number of test strips/specimens
   Mean value of the mean delamination force in the transverse direction and in the machine direction
   Mean value of maximum values
   Mean value of minimum values
   Standard deviation
Specification of separation code list (Example: 2 A1 +3B)
Accuracy: 0.01 N
Reference: ASTM D 1876-72

The mechanical tape fastener 25,26 in accordance with the present invention is attached in folded condition to the inner face of the inner layer 4. The manufacturing process may therefore be performed in a simpler and more reliable manner than in the case of the prior art solutions, such as a solution involving Y-tapes. As mentioned above, all folding steps with regard to the mechanical tape fasteners for the article, in accordance with the present invention may be performed at a considerable lower speed than at the high web velocities found in modern machines for rational and competitive production of disposable absorbent articles, such as baby diapers.

The manufacturing of the mechanical tape fasteners in accordance with the present invention can be performed at such a first, lower speed which is only about 5-30 % of a second, higher speed at which the diapers are produced.

The process for manufacturing mechanical tape fasteners includes the following steps.:
A. A carrier web, for instance of a three-ply configuration as has been described in connection with the embodiment shown in figure 5, i.e. an intermediate ply in the form of an elastic film, a non-woven ply and a non-elastic ply of a non-woven, is supplied to a manufacturing line for the mechanical tape fasteners.
B. A longitudinal edge part of the carrier web is folded over itself.
C. The folded edge part is attached to the unfolded part by means of a weak, breakable bond, preferably in the form of thermal or ultrasonic welds. The bond can be in the form of a pattern of linear welds or spaced apart dots. The bonds can represent a part of a pattern or be randomly distributed.
D. In case two rows of carrier webs are formed out of the same base material the carrier web is divided.
E. Diaper user attachment means in the form of a web with projecting hooks, intended to form one part of a hook-and-loop fastener are joined to the unfolded parts.
F. Diaper manufacturer attachment means, in the form of glue are joined to the folded part of the carrier web so that both user and manufacturer attachment means are facing at the same direction.
G. Diaper fastening tabs are formed by cutting the tab carrier material perpendicular to the travelling direction.
H. The diaper fastening tabs, which have been formed at a first low speed, are accelerated from the first, low speed to a second, higher speed, corresponding to the manufacturing speed of the diapers.
I. The diaper fastening tabs are attached at said second, higher speed to the upward facing side of the diaper material web forming the inner layer of the diapers.
   An attached diaper fastening tab is shown in figure 3. The outer edge portion 33 externally of the fastening part 31 could possibly be provided with a pressure sensitive adhesive (not shown) when the fastening part with hooks are fastened to the carrier web in step E above.

The invention is not limited to the examples described above but several modifications are possible within the scope of the appended claims.

The elastic tape fastener may comprise several folds when in its storage condition. This may be appropriate if longer tape fasteners are desired, for example when it is desirable to give the diaper itself a more narrow shape. It is, however essential that the number of folds is an odd number to allow the tape fasteners to be attached permanently to the inner layer and by means of the mechanical tape bonding face be joined to the complementary bonding face on the outer face of the outer layer.

The tape fastener naturally need not be designed with three plies. The internal non-woven ply 35, for instance, may be dispensed with.

In addition, also the corrugated non-woven ply may be eliminated. One example of a suitable material of this kind is an elastic film marketed by Tredegar under the name Fabriflex 106D.

The tape fastener need not either include an elastic part. A suitable non-elastic alternative is a spunbond non-woven product having a weight per unit of 40-100 g/m² marketed by Pegas

The above description has been made with reference to a baby diaper. However, the present invention embraces also adults' diapers and other sanitary disposable absorbent articles that may be attached about the waist of the wearer, such as women's sanitary diaperss. The width of the mechanical tape fastener may be in the order of 40mm. If longer tape fasteners are desired, the width may be increased for more stability. The width of the tape fasteners should be in the range of 30 to 100mm.

## Claims

1. A disposable absorbent article, such as a baby diaper, an incontinence diaper or the like, comprising a first end portion (1) and a second end portion (2), which portions are intended completely or partly to enclose the user's waist area during use of the article, and an intermediate crotch portion (3), said portions comprising an inner layer (4), which is turned towards the user during use and which is liquid-permeable at least in the crotch portion opposite the user's genitals, a liquid-impermeable outer layer (14), and an absorbent body (21) disposed between said outer and inner layers, said article being formed in the area of the second end portion (2) with mechanical tape fasteners (25,26) intended, upon application of the article, to be detachably interconnected with one or several fastening parts (27) disposed on the outer layer of the first end portion (1) and being complementary to said tape fasteners, **characterized in**
**that** the article comprises at least two of said tape fasteners (25,26), which are arranged on the inner layer (4) of the article at opposite side portions (23,24) of said second end portion (2),
**that** the tape fasteners (25,26) have one longitudinal and one transversal extension,
**that** said tape fasteners are permanently joined to the inner layer (4) of the article by means of a producer's bonding face (39) arranged on a first end part (28) of the tape fasteners (25,26),
**that** a user's bonding face (31) is arranged on the opposite second end part (30) of the tape fasteners, the user's end part,
the tape fasteners (25,26) are arranged in a folded storage condition, each one of said tape fasteners being formed with an odd number of folds (38) and the bonding faces of both said producer's bonding face (39) and said user's bonding face (31) being turned towards the inner layer (4) of the article,
**that** the user's end parts (30) of two opposite tape fasteners (25,26) face one another in said storage condition and that in the storage condition of said article individual fold parts (28 and 29, respectively) are joined to at least one of the other fold parts (28 and 29, respectively) by means of a breakable bond (32), in the form of thermal or ultrasonic welds, said bond being breakable in order to establish the position of use of said tape fasteners.

2. An article as claimed in claim 1, **characterized in that** the tape fasteners (25,26) are arranged in their entirety on the inner layer (4), with the laterally outermost end edges (38) of the tape fasteners being located interiorly of the lateral edges of said lateral portions (23,24).

3. An article as claimed in claim 1 or 2, **characterized in that** the mechanical tape fasteners (25,26) comprise one fold (38) only and **in that** the latter forms the laterally outermost end edge of the tape fastener in said storage condition, that the tape fasteners comprise a middle part (29), which by means of said breakable bond (32) is connected to the first end part (28) and which covers the latter, and **in that** when the user's bonding face (31) is in said folded storage condition, the opposite end part (30) is located laterally interiorly of the first end part (28).

4. An article as claimed in claim 3, **characterized in that** the user's bonding face (31) is in the form of hooks of a hook-and-loop fastener and **in that** the complementary fastening part (27) forms the other half of the hook-and-loop fastener and is made from a material, in which the hooks are able to fasten, said material being formed with apertures, loops or fibre filaments for co-operation with the hooks.

5. An article as claimed in claim 4, **characterized in that** the inner layer (4) of the article comprises fibres in which the hooks of the user's bonding face (31) fasten to form a weak bond relatively to the hook-and-loop fastener and said bond between the middle part and the first end part.

6. An article as claimed in any one of claims 3-5, **characterized in**
**that** the outermost end part (33) of the mechanical tape fastener (25) is coated with a pressure-sensitive adhesive providing a weak adhesion force.

7. An article as claimed in any one of the preceding claims, **characterized in**
**that** said breakable bond (32) is designed to ensure that a force in the range of 0,2-2N, preferably in the range of 0.5-1.5N, is required to separate from one another joined-together tape-fastener strips having a width of 40mm.

8. An article as claimed in any one of the preceding claims, **characterized in**
**that** the bond (32) is in the form of a pattern of points or lines, and in that the bond is breakable without damage to the tape fastener (25).

9. An article as claimed in any one of the preceding claims, **characterized in**
**that** the mechanical tape fasteners (25,26) have a width of between 30 and 10 mm.

10. An article as claimed in any one of the preceding claims, **characterized in**
**that** the mechanical tape fasteners comprise at least one elastic part (29) disposed between the two end parts (28,30), said elastic part arranged to stretch upon occurrence of load during use of the article.

11. An article as claimed in any one of claims 1-9, **characterized in**
**that** the mechanical tape fasteners (25, 26) do not stretch upon occurrence of load during use of the article.

## Patentansprüche

1. Absorbierender Einweg-Gegenstand wie beispielsweise eine Babywindel, eine Inkontinenzwindel oder Ähnliches, umfassend einen ersten Endabschnitt (1) und einen zweiten Endabschnitt (2), welche Abschnitte dazu gedacht sind, im Gebrauch des Gegenstandes den Taillenbereich eines Benutzers vollständig oder teilweise zu umschließen, und einen dazwischen liegenden Schrittabschnitt (3), wobei die Abschnitte eine Innenlage (4), die im Gebrauch in Richtung des Benutzers gewandt ist und wenigstens im Schrittabschnitt gegenüber den Genitalien des Benutzers flüssigkeitsdurchlässig ist, eine flüssigkeitsundurchlässige Außenlage (14) und einen zwischen der Außen- und Innenlage angeordneten Absorptionskörper (21) umfassen, wobei der Gegenstand im Bereich des zweiten Endabschnitts (2) mit mechanischen Streifenverschlüssen (25, 26) ausgebildet ist, die dazu gedacht sind, beim Anlegen des Gegenstandes lösbar mit einem oder mehreren Verschlussteilen (27), die auf der Außenlage des ersten Endabschnitts (1) angeordnet sind und die komplementär zu den Streifenverschlüssen sind, verbunden zu werden, **dadurch gekennzeichnet, dass**
der Gegenstand wenigstens zwei Streifenverschlüsse (25, 26) umfasst, die auf der Innenlage (4) des Gegenstandes an entgegengesetzten Seitenabschnitten (23, 24) des zweiten Endabschnitts (2) angeordnet sind,
dass die Streifenverschlüsse (25, 26) eine Längs- und eine Quererstreckung aufweisen,
dass die Streifenverschlüsse mittels einer Hersteller-Verbindungsfläche (39), die auf einem ersten Endteil (28) der Streifenverschlüsse (25, 26) angeordnet ist, permanent mit der Innenlage (4) des Gegenstandes verbunden sind,
dass eine Benutzer-Verbindungsfläche (31) auf dem entgegengesetzten zweiten Endteil (30) der Streifenverschlüsse, dem Benutzer-Endteil angeordnet ist,
die Streifenverschlüsse (25, 26) in einem gefalteten Lagerzustand angeordnet sind, wobei die Streifenverschlüsse jeweils mit einer ungeraden Anzahl an Falten (38) ausgebildet sind und die Verbindungsflächen von sowohl der Hersteller-Verbindungsfläche (39) als auch der Benutzer-Verbindungsfläche (31) in Richtung der Innenlage (4) des Gegenstandes gewandt sind,
dass die Benutzer-Endteile (30) der zwei entgegengesetzten Streifenverschlüsse (25, 26) im Lagerzustand einander zugewandt sind und dass im Lagerzustand des Gegenstandes einzelne Faltteile (28 bzw. 29) mit wenigstens einem der anderen Faltteile (28 bzw. 29) mittels einer aufbrechbaren Verbindung (32) in Form einer thermischen oder einer Ultraschall-Schweißnaht verbunden sind, wobei die Verbindung aufbrechbar ist, um die Gebrauchsposition der Streifenverschlüsse zu bewirken.

2. Gegenstand nach Anspruch 1, **dadurch gekennzeichnet, dass** die Streifenverschlüsse (25, 26) in ihrer Gesamtheit auf der Innenlage (4) angeordnet sind, wobei die seitlich äußersten Endkanten (38) der Streifenverschlüsse innerhalb der Seitenkanten der Seitenabschnitte (23, 24) angeordnet sind.

3. Gegenstand nach Anspruch 1 oder 2, **dadurch gekennzeichnet, dass** die mechanischen Streifenverschlüsse (25, 26) nur eine Falte (38) umfassen und **dadurch**, dass letzterer die seitlich äußerste Endkante der Streifenverschlüsse im Lagerzustand bildet, dass die Streifenverschlüsse einen Mittelteil (29) umfassen, der mittels der aufbrechbaren Verbindung (32) mit dem ersten Endteil (28) verbunden ist und letzteren bedeckt und **dadurch**, dass wenn die Benutzer-Verbindungsfläche (31) in dem besagten gefalteten Lagerzustand vorliegt, der entgegengesetzte Endteil (30) seitlich innerhalb des ersten Endteils (28) angeordnet ist.

4. Gegenstand nach Anspruch 3, **dadurch gekennzeichnet, dass** die Benutzer-Verbindungsfläche (31) in Form von Haken eines Haken- und Schlaufenverschlusses vorliegt und **dadurch**, dass der komplementäre Verschlussteil (27) die andere Hälfte des Haken- und Schlaufenverschlusses bildet und aus einem Material gebildet ist, an dem die Haken befestigt werden können, wobei das Material mit Öffnungen, Schlaufen oder Faserfilamenten zum Zusammenwirken mit den Haken versehen ist.

5. Gegenstand nach Anspruch 4, **dadurch gekennzeichnet, dass** die Innenlage (4) des Gegenstandes Fasern umfasst, an denen sich die Haken der Benutzer-Verbindungsfläche (31) befestigen, um relativ zu dem Haken- und Schlaufenverschluss und der Verbindung zwischen dem Mittelteil und dem ersten Endteil eine schwache Verbindung zu bilden.

6. Gegenstand nach einem der vorstehenden Ansprüche 3 bis 5, **dadurch gekennzeichnet, dass** der äußerste Endteil (33) des mechanischen Streifenverschlusses (25) mit einem Haftklebemittel beschichtet ist, das eine schwache Haftkraft bereitstellt.

7. Gegenstand nach einem der vorstehenden Ansprüche, **dadurch gekennzeichnet, dass** die aufbrechbare Verbindung (32) ausgestaltet ist um zu sichern, dass eine Kraft im Bereich von 0,2-2 N, vorzugsweise im Bereich von 0,5-1,5N erforderlich ist, um miteinander verbundene Streifenverschlüsse mit einer Breite von 40 mm voneinander zu trennen.

8. Gegenstand nach einem der vorstehenden Ansprüche, **dadurch gekennzeichnet, dass** die Verbindung (32) in Form eines Musters aus Punkten oder Linien vorliegt und **dadurch**, dass die Verbindung ohne Beschädigung des Streifenverschlusses (25) aufbrechbar ist.

9. Gegenstand nach einem der vorstehenden Ansprüche, **dadurch gekennzeichnet, dass** die mechanischen Streifenverschlüsse (25, 26) eine Breite zwischen 30 und 10 mm aufweisen.

10. Gegenstand nach einem der vorstehenden Ansprüche, **dadurch gekennzeichnet, dass** die mechanischen Streifenverschlüsse wenigstens einen elastischen Teil (29) umfassen, der zwischen den zwei Endteilen (28, 30) angeordnet ist, wobei der elastische Teil angeordnet ist, um sich im Gebrauch auf das Auftreten einer Last des Gegenstandes zu dehnen.

11. Gegenstand nach einem der Ansprüche 1 bis 9, **dadurch gekennzeichnet, dass** sich die mechanischen Streifenverschlüsse (25, 26) beim Auftreten einer Last im Gebrauch des Gegenstandes nicht dehnen.

## Revendications

1. Article absorbant jetable, tel qu'une couche-culotte, une couche pour l'incontinence ou similaire, comprenant une première portion d'extrémité (1) et une deuxième portion d'extrémité (2), lesquelles portions sont destinées à recouvrir complètement ou partiellement la région de taille de l'utilisateur pendant l'utilisation de l'article, et une portion d'entrejambe intermédiaire (3), lesdites portions comprenant une couche intérieure (4), qui est tournée vers l'utilisateur pendant l'utilisation et qui est perméable aux liquides au moins dans la portion d'entrejambe, en face des parties génitales de l'utilisateur, une couche extérieure (14) imperméable aux liquides et un corps absorbant (21) placé entre lesdites couches extérieure et intérieure, ledit article étant muni, dans la région de la deuxième portion d'extrémité (2), d'éléments de fermeture en ruban mécaniques (25, 26) destinés, lors de l'application de l'article, à être interconnectés de manière détachable avec une ou plusieurs pièces de fixation (27) placées sur la couche extérieure de la première portion d'extrémité (1) et complémentaires desdits éléments de fermeture en ruban, **caractérisé en ce que** :
l'article comprend au moins deux desdits éléments de fermeture en ruban (25, 26), qui sont disposés sur la couche intérieure (4) de l'article en des parties latérales opposées (23, 24) de ladite deuxième portion d'extrémité (2),
les éléments de fermeture en ruban (25, 26) ont une dimension longitudinale et une dimension transversale,
lesdits éléments de fermeture en ruban sont liés de façon permanente à la couche intérieure (4) de l'article au moyen d'une face de collage d'un producteur (39) placée sur une première partie d'extrémité (28) des éléments de fermeture en ruban (25, 26),
une face de collage d'utilisateur (31) est placée sur la deuxième partie d'extrémité, opposée, (30) des éléments de fermeture en ruban, la partie d'extrémité de l'utilisateur,
les éléments de fermeture en ruban (25, 26) sont agencés dans un état de stockage plié, chacun desdits éléments de fermeture en ruban étant pourvu d'un nombre impair de plis (38) et les faces de collage de ladite face de collage de producteur (39) et de ladite face de collage d'utilisateur (31) étant tournées vers la couche intérieure (4) de l'article,
les parties d'extrémité d'utilisateur (30) de deux éléments de fermeture en ruban opposés (25, 26) se font face dans ledit état de stockage et dans l'état de stockage dudit article des parties pliées individuelles (28 et 29, respectivement) sont liées à au moins l'une des autres parties pliées (28 et 29, respectivement) au moyen d'un lien cassable (32), sous forme de soudures thermiques ou par ultrasons, ledit lien pouvant être rompu afin d'établir la position d'utilisation desdits éléments de fermeture en ruban.

2. Article selon la revendication 1, **caractérisé en ce que** les éléments de fermeture en ruban (25, 26) sont intégralement situés sur la couche intérieure (4), les bords d'extrémité latéralement les plus à l'extérieur (38) des éléments de fermeture en ruban étant situés à l'intérieur des bords latéraux desdites parties latérales (23, 24).

3. Article selon la revendication 1 ou 2, **caractérisé en ce que** les éléments de fermeture en ruban mécaniques (25, 26) comprennent un pli (38) seulement et **en ce que** ce dernier forme le bord d'extrémité latéralement le plus à l'extérieur de l'élément de fermeture en ruban dans ledit état de stockage, **en ce que** les éléments de fermeture en ruban comprennent une partie médiane (29), qui, par le biais dudit lien cassable (32), est connectée à la première partie d'extrémité (28) et qui couvre cette dernière, et **en ce que**, lorsque la face de collage de l'utilisateur (31) est dans ledit état de stockage plié, la partie d'extrémité opposée (30) est située latéralement à l'intérieur de la première partie d'extrémité (28).

4. Article selon la revendication 3, **caractérisé en ce que** la face de collage de l'utilisateur (31) se présente sous la forme de crochets d'un élément de fermeture à crochets et à boucles et **en ce que** la pièce de fixation complémentaire (27) forme l'autre moitié de l'élément de fermeture à crochets et à boucles et est faite d'un matériau, dans lequel les crochets peuvent se fixer, ledit matériau étant muni d'ouvertures, de boucles ou de filaments de fibre destiné(e)s à coopérer avec les crochets.

5. Article selon la revendication 4, **caractérisé en ce que** la couche intérieure (4) de l'article comprend des fibres dans lesquelles les crochets de la face de collage de l'utilisateur (31) se fixent pour former une liaison faible par rapport à l'élément de fermeture à boucles et crochets et à ladite liaison entre la partie médiane et la première partie d'extrémité.

6. Article selon l'une quelconque des revendications 3 à 5, **caractérisé en ce que** la partie d'extrémité la plus extérieure (33) de l'élément de fermeture en ruban mécanique (25) est revêtue d'un adhésif sensible à la pression qui fournit une force d'adhésion faible.

7. Article selon l'une quelconque des revendications précédentes, **caractérisé en ce que** ledit lien cassable (32) est conçu pour assurer qu'une force comprise dans l'intervalle de 0,2 à 2 N, de préférence de 0,5 à 1,5 N, est nécessaire pour séparer l'une de l'autre des bandes de fermeture à ruban liées ensemble ayant une largeur de 40 mm.

8. Article selon l'une quelconque des revendications précédentes, **caractérisé en ce que** le lien (32) se présente sous la forme d'un motif constitué de points ou de lignes, et **en ce que** le lien peut être rompu sans endommager l'élément de fermeture en ruban (25).

9. Article selon l'une quelconque des revendications précédentes, **caractérisé en ce que** les éléments de fermeture en ruban mécaniques (25, 26) ont une largeur comprise entre 30 et 10 mm.

10. Article selon l'une quelconque des revendications précédentes, **caractérisé en ce que** les éléments de fermeture en ruban mécaniques comprennent au moins une partie élastique (29) placée entre les deux parties d'extrémité (28, 30), ladite partie élastique étant agencée pour s'étirer lors de l'apparition d'une charge pendant l'utilisation de l'article.

11. Article selon l'une quelconque des revendications 1 à 9, **caractérisé en ce que** les éléments de fermeture en ruban mécaniques (25, 26) ne s'étirent pas lors de l'apparition d'une charge pendant l'utilisation de l'article.
